## Europäisches Patentamt

## European Patent Office

## Office européen des brevets

(11) Veröffentlichungsnummer: **0 048 949**
**A1**

(12) **EUROPÄISCHE PATENTANMELDUNG**

(21) Anmeldenummer: **81107566.2**

(22) Anmeldetag: **23.09.81**

(51) Int. Cl.³: **C 02 F 9/00**, C 02 F 1/52,
C 02 F 1/20, G 01 N 33/18

(30) Priorität: **26.09.80 DE 3036285**

(71) Anmelder: **HOECHST AKTIENGESELLSCHAFT,
Postfach 80 03 20, D-6230 Frankfurt/Main 80 (DE)**

(43) Veröffentlichungstag der Anmeldung: **07.04.82
Patentblatt 82/14**

(72) Erfinder: **Melzer, Werner, Dr., Drosselweg 2,
D-6237 Liederbach (DE)**
Erfinder: **Weidmann, Karl, Hauptstrasse 24, D-6232 Bad
Soden am Taunus (DE)**
Erfinder: **Kühmichel, Horst, Von-Eichendorff-Strasse 17,
D-6384 Schmitten/Taunus (DE)**

(84) Benannte Vertragsstaaten: **AT BE CH DE FR GB IT LI NL
SE**

(54) Verfahren und Vorrichtung zum Aufbereiten von Abwasser für analysentechnische Zwecke.

(57) Bei dem Verfahren zum Aufbereiten von Abwasser für analysentechnische Zwecke durch Sedimentation und Filtration wird das Abwasser einer ersten Sedimentationsstufe (3) zugeleitet. Dieser Vorgang wird mindestens einmal wiederholt, wobei jeweils das an festen Wasserinhaltsstoffen verarmte Abwasser der nächsten Sedimentationsstufe (8, 9) zugeleitet wird. In Anschluss an die letzte Sedimentationsstufe (9) lässt man das an festen Wasserinhaltsstoffen verarmte Abwasser eine Bandfiltereinrichtung (13) passieren. Es kann dort als aufbereitetes Wasser für Analysezwecke entnommen werden. Nach der Entnahme des Wassers wird die Aufbereitungsanlage entleert. Danach kann die Aufbereitung erneut beginnen.

0048949

HOECHST AKTIENGESELLSCHAFT    HOE 80/F 217    D.Ph.HS/ sch

Verfahren und Vorrichtung zum Aufbereiten von Abwasser für
analysentechnische Zwecke

Gegenstand der Erfindung ist ein Verfahren und eine Vorrichtung zum Aufbereiten von Abwasser für analysentechnische
Zwecke durch Sedimentation und Filtration.

Das mit Prozessanalysatoren zu untersuchende Abwasser muß
vor Eintritt in die entsprechenden Analysengeräte aufbereitet werden. Dazu gehört die Abtrennung von festen Wasserinhaltsstoffen, sowie gegebenenfalls die Abtrennung des
anorganisch gebundenen Kohlenstoffs im Falle der Messung
des organisch gebundenen Kohlenstoffs.

Die Aufgabe wird durch ein Verfahren gelöst, das dadurch
gekennzeichnet ist, daß man das Abwasser einer ersten
Sedimentationsstufe zuleitet, das an festen Wasserinhaltsstoffen verarmte Abwasser dieser Stufe der nächsten Sedimentationsstufe zuleitet, nach der letzten Sedimentationsstufe das an festen Wasserinhaltsstoffen verarmte Abwasser
einer Bandfiltereinrichtung passieren läßt und dort als
aufbereitetes Abwasser entnimmt, nach der Entnahme des Abwassers die Aufbereitungsanlage entleert und den Aufbereitungsvorgang erneut beginnt.

Zum Austreiben des anorganisch gebundenen Kohlenstoffs
kann das Abwasser der letzten Sedimentationsstufe angesäuert
und mit Luft behandelt werden.

Eine Vorrichtung zum Durchführen des Verfahrens zum Aufarbeiten von Abwasser kann beispielsweise dadurch gekennzeichnet sein, daß mehrere durch Leitungen verbundene Sedimentationsbehälter je einen Überlauf und einen Bodenauslauf
aufweisen, darüberhinaus der erste Behälter mit einem
Zulauf für das Abwasser und der letzte Behälter mit einer
Entnahmeeinrichtung für das aufbereitete Abwasser versehen ist, die auf der Klarlaufseite einer im oberen Teil dieses Behälters befindlichen, beweglichen Bandfiltereinrichtung angeordnet ist.

Im Zulauf, in den Verbindungsleitungen und in den Bodenausläufen können mit einer Steuereinrichtung verbundene Ventile angeordnet sein. In den letzten Sedimentationsbehälter kann eine Leitung für Säure einmünden, die mit einem Ventil versehen ist, das über eine pH-Meß- und Regeleinrichtung mit einer im letzten Behälter angeordneten pH-Meßkette verbunden ist. Ferner kann im letzten Sedimentationsbehälter eine Begasungseinrichtung angeordnet sein.

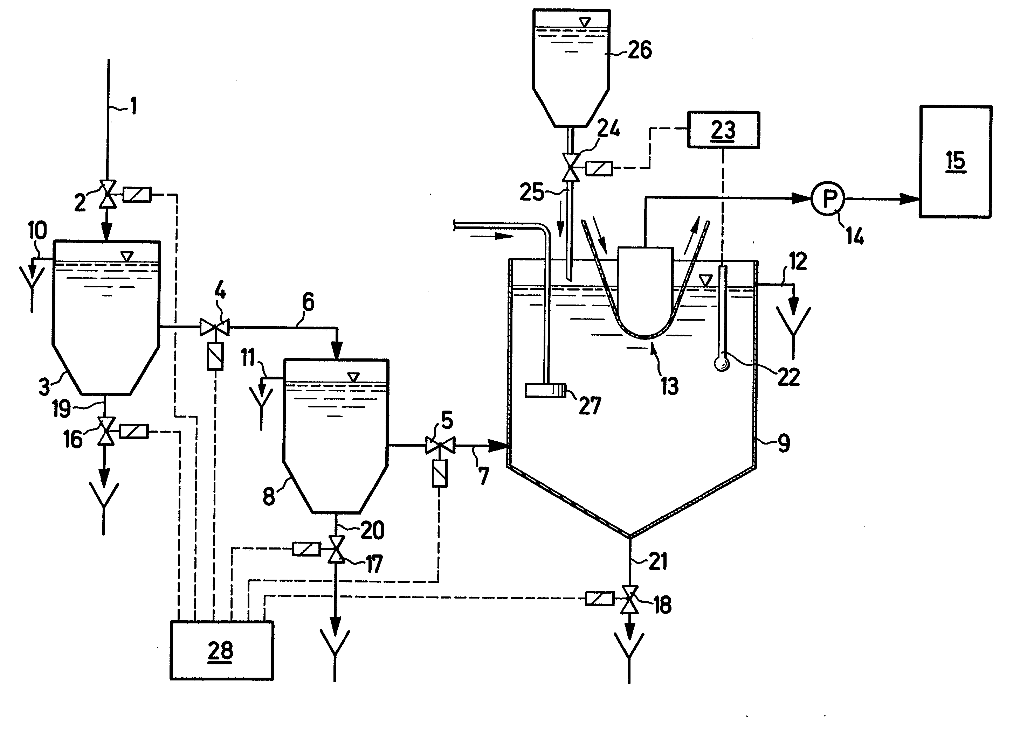

Im Folgenden wird die Erfindung anhand der Figur, die ein Verfahrensschema darstellt, näher erläutert.

Das analysentechnisch, z.B. gaschromatographisch, kalorimetrisch, colorimetrisch oder photometrisch zu untersuchende Abwasser wird über Leitung 1 und zeitgesteuertem Ventil 2 einem Sedimentationsbehälter 3 zugeleitet. Das an festen Wasserinhaltsstoffen verarmte Abwasser gelangt über ebenfalls mit zeitgesteuerten Ventilen 4 und 5 versehene Leitungen 6 und 7 in weitere Sedimentationsbehälter 8 und 9. Die flotierten festen Wasserinhaltsstoffe werden über Überläufe 10, 11 und 12 abgeführt. Im letzten Sedimentationsbehälter 9 ist eine bewegliche Bandfiltereinrichtung 13, z.B. ein sich selbst erneuerndes Papierfilter angeordnet, über das das aufbereitete Abwasser mittels einer Entnahmeeinrichtung 14, z.B. einer Pumpe, entnommen und dem Analysator 15 zugeführt wird. Während der Entnahme des Abwassers oder nach erfolgter Entnahme werden die zeitgesteuerten Ventile 2, 4 und 5 geschlossen, und die ebenfalls zeitgesteuerten Ventile 16, 17 und 18 in den Bodenausläufen 19, 20 und 21 geöffnet, um die Aufbereitungsanlage zu entleeren und für eine neue Abwasseraufbereitung vorzubereiten. Die Ventile 2, 4, 5, 16, 17 und 18 können mit einer elektrischen, pneumatischen, hydraulischen oder mechanischen Steuereinheit 28 verbunden sein. Soll der im Abwasser organisch gebundene Kohlenstoffgehalt bestimmt werden, muß zuvor der anorganisch gebundene Kohlenstoff entfernt werden. Das erfindungsgemäße Verfahren eignet sich in besonders vorteilhafter Weise hierfür. Der Inhalt des letzten Sedimentationsbehälters wird angesäuert. Zu diesem Zweck ist eine pH-Meßkette 22 im Behälter 9 angeordnet, mit deren Hilfe über eine pH-Meß- und Regeleinrichtung 23 ein Ventil 24 im Zulauf 25 für Säure betätigt wird. 26 deutet einen Vorratsbehälter für die Säure an. 27 soll eine Begasungseinrichtung symbolisieren. Mit dem Gas, z.B. Luft, wird das Kohlendioxid ausgetragen.

HOE 80/F 217

00 48949

Patentansprüche:

1. Verfahren zum Aufbereiten von Abwasser für analysentechnische Zwecke durch Sedimentation und Filtration, dadurch gekennzeichnet, daß man das Abwasser einer ersten Sedimentationsstufe zuleitet, das an festen Wasserinhaltsstoffen verarmte Abwasser dieser Stufe der nächsten Sedimentationsstufe zuleitet, nach der letzten Sedimentationsstufe das an festen Wasserinhaltsstoffen verarmte Abwasser eine Bandfiltereinrichtung passieren läßt und dort als aufbereitetes Wasser entnimmt, nach der Entnahme des Abwassers die Aufbereitungsanlage entleert und den Aufbereitungsvorgang erneut beginnt.

2. Verfahren nach Anspruch 1, dadurch gekennzeichnet, daß man das Abwasser der letzten Sedimentationsstufe ansäuert und mit Luft behandelt.

3. Vorrichtung zum Aufbereiten von Abwasser für analysentechnische Zwecke durch Sedimentation und Filtration, dadurch gekennzeichnet, daß mehrere durch Leitungen (6, 7) verbundene Sedimentationsbehälter (3, 8, 9) je einen Überlauf (10, 11, 12) und einen Bodenauslauf (19, 20, 21) aufweisen, darüberhinaus der erste Behälter (3) mit einem Zulauf (1) für das Abwasser und der letzte Behälter (9) mit einer Entnahmeeinrichtung (14) für das aufbereitete Abwasser versehen ist, die auf der Klarlaufseite einer im oberen Teil dieses Behälters befindlichen, beweglichen Bandfiltereinrichtung (13) angeordnet ist.

4. Vorrichtung nach Anspruch 3, dadurch gekennzeichnet, daß im Zulauf (1), in den Verbindungsleitungen (6, 7) und in den Bodenausläufen (19, 20, 21) mit einer Steuereinrichtung (28) verbundene Ventile (16, 17, 18) angeordnet sind.

5. Vorrichtung nach den Ansprüchen 3 und 4, dadurch gekennzeichnet, daß in den letzten Sedimentationsbehälter (9) eine Leitung (25) für Säure einmündet, die mit einem Ventil (24) versehen ist, das über eine pH-Meß- und Regeleinrichtung (23) mit einer im letzten Behälter (9) angeordneten pH-Meßkette (22) verbunden ist.

6. Vorrichtung nach den Ansprüchen 3, 4 oder 5, dadurch gekennzeichnet, daß im letzten Sedimentationsbehälter (9) eine Begasungseinrichtung (27) angeordnet ist.

| Kategorie | Kennzeichnung des Dokuments mit Angabe, soweit erforderlich, der maßgeblichen Teile | betrifft Anspruch |
|---|---|---|
| | DE - A1 - 2 819 153 (WACKER-CHEMIE) <br><br> * Anspruch 1 * <br><br> -- | 1 |
| | DE - A1 - 2 806 109 (HAKANSSON) <br><br> * Seite 3, Zeilen 1-7; Seite 4, Zeilen 1-24 * <br><br> -- | 1 |
| | AT - B - 223 142 (GEIGER) <br><br> * Seite 1, Zeilen 11-23 * <br><br> -- | 1 |
| | DE - B2 - 2 060 620 (PASSAVANT-WERKE) <br><br> * Spalte 4, Zeilen 10-33 * <br><br> -- | 1,3 |
| | DE - A1 - 2 401 862 (GEIGER) <br><br> * Seite 1, Zeilen 1-4 * <br><br> ---- | 1 |

**EINSCHLÄGIGE DOKUMENTE**

**KLASSIFIKATION DER ANMELDUNG (Int. Cl.)**

C 02 F 9/00
C 02 F 1/52
C 02 F 1/20
G 01 N 33/18

**RECHERCHIERTE SACHGEBIETE (Int. Cl.)**

C 02 F
G 01 N

**KATEGORIE DER GENANNTEN DOKUMENTE**

X: von besonderer Bedeutung
A: technologischer Hintergrund
O: nichtschriftliche Offenbarung
P: Zwischenliteratur
T: der Erfindung zugrunde liegende Theorien oder Grundsätze
E: kollidierende Anmeldung
D: in der Anmeldung angeführtes Dokument
L: aus andern Gründen angeführtes Dokument
&: Mitglied der gleichen Patentfamilie, übereinstimmendes Dokument

X | Der vorliegende Recherchenbericht wurde für alle Patentansprüche erstellt.

| Recherchenort | Abschlußdatum der Recherche | Prüfer |
|---|---|---|
| WIEN | 16-12-1981 | WILFLINGER |

EPA form 1503.1  06.78